Europäisches Patentamt

European Patent Office

Office européen des brevets

(11) Veröffentlichungsnummer: **0 426 969 A2**

## (12) EUROPÄISCHE PATENTANMELDUNG

(21) Anmeldenummer: 90117254.4

(22) Anmeldetag: 07.09.90

(51) Int. Cl.5: **A61N 1/08**

(30) Priorität: 10.11.89 DE 3937552
25.01.90 DE 4002187

(43) Veröffentlichungstag der Anmeldung:
**15.05.91 Patentblatt 91/20**

(84) Benannte Vertragsstaaten:
**DE FR GB IT**

(71) Anmelder: **LEWICKI MICROELECTRONIC GmbH**
**Allee 35**
**W-7938 Oberdischingen b. Ulm(DE)**

(72) Erfinder: **Colen, Fred A., Dipl.-Ing.**
**Albstrasse 76**
**W-7930 Ehingen(DE)**

(74) Vertreter: **Patentanwälte Deufel- Schön-
Hertel- Lewald**
**Isartorplatz 6**
**W-8000 München 2(DE)**

(54) **Schutzschaltung.**

(57) Zur Verbesserung der Störfestigkeit eines therapeutisch und/oder diagnostisch wirksamen Gerätes, insbesondere eines Herzschrittmachers mit Eingangskreis (10) und Ausgangskreis (12) ist eine Abkopplungsschaltung (20) vorgesehen, so daß der Ausgangskreis (12) von dem Anschlußklemmenpaar (16, 18) des Gerätes abgekoppelt ist. Die Abkopplung des unsymmetrisch begrenzenden Ausgangskreises (12) erfolgt immer und unabhängig von evtl. vorhandenen Störsignalen für die gesamte Impulsperiodendauer.

FIG.1

Die Erfindung betrifft eine Schutzschaltung zur Beseitigung des Einflusses von kontinuierlichen, gepulsten oder modulierten Störsignalen für therapeutisch und/oder diagnostisch wirksame Geräte.

Herzschrittmacher erkennen über eine angeschlossene Elektrode Herzeigenaktionen. Diese elektrischen Signale liegen im Niederfrequenzbereich (bis max. 1 kHz) und haben eine Amplitude von 1 bis 10 mV. Herzschrittmacher dürfen durch Einwirkung einer Vielfalt von Störsignalen in ihrer bestimmungsgemäß therapeutischen Tätigkeit nicht beeinflußt werden.

Es sind eine Vielzahl von Schutzschaltungen bekannt, um den Einfluß von Störsignalen bei einem Herzschrittmacher zu reduzieren. Eine Lösung besteht darin, am Anschlußklemmenpaar eines Herzschrittmachers einen Kondensator anzuordnen, der die höher frequenten Störsignale dämpft. Bei einer anderen Lösung ist bei einem Herzschrittmacher nach dem Eingangsfilter und -verstärker eine elektronische Schaltung vorgesehen, die die Umschaltung auf eine 100%-ige Herzstimulation bewirkt, wenn ein kontinuierlich vorhandenes Störsignal vorliegt. Dabei geht aber die Wahrnehmung von Herzeigenaktionen verloren.

Eine weiterführende Schaltungsvariante ermöglicht die Wahrnehmung von Herzeigenaktionen und vermeidet die Umschaltung auf 100%-ige Stimulation bei kontinuierlichen Störungen.

Die heute vorhandenen Herzschrittmacher werden durch die vorstehend beschriebenen Schaltungsvarianten nicht ausreichend vor vorhandenen gepulsten oder modulierten Störsignalen geschützt. Derartige Störsignale können daher zu einer Unterdrückung therapeutisch notwendiger Herzschrittmacherstimulationsimpulse führen. Diese Störsignale werden durch die bekannten Schaltungen lediglich durch den Eingangkondensator und mögliche weitere interne Filter gedämpft.

Das Institut für Rundfunktechnik GmbH, München, berichtet in EMV '88 545-554 über die "Beeinflussung von Herzschrittmachern durch leistungsstarke Funksender". Darin wird eine Störbeeinflussung an 34 verschiedenen Schrittmachertypen veröffentlicht. Die Ergebnisse zeigen z.B. eine Schrittmacherbeeinflussung bei einer Störspannung von 0,05 V bis 2 V bei 30 kHz und bei einer Störspannung von 0,2 V bis 17 V bei 500 kHz.

Die Anforderungen an zeitgemäße Schrittmacher, was den Störschutz betrifft, werden immer höher. Dies zeigt sich auch in den Diskussionen bei den DIN/VDE- und CENELEC Normungsarbeiten. Das IRT München fordert beispielsweise eine Herzschrittmacher-Störspannungsfestigkeit von mindestens 16 V, auch für modulierte und gepulste Störsignale im Frequenzbereich von 30 kHz bis 30 MHz.

Der Erfindung liegt die Aufgabe zugrunde, eine Schutzschaltung zu schaffen, durch welche mit einfachen Mitteln die heutzutage geforderte Störspannungsfestigkeit erreicht wird.

Diese Aufgabe wird erfindungsgemäß dadurch gelöst, daß zwischen Ausgangskreis und dem Anschlußklemmenpaar eine Abkopplungsschaltung angeordnet ist, die eine erste Serienschaltung aus einer Diode und der Kollektor-Emitterstrecke eines Transistors zwischen Ausgangskreis und einer Anschlußklemme erhält, wobei die Basis des Transistors über einen Basiswiderstand mit der anderen Anschlußklemme verbunden ist.

Gemäß einer bevorzugten Ausführungsform ist zwischen den beiden Anschlußklemmen eine zweite Serienschaltung aus einer Diode und der Kollektor-Emitterstrecke eines zweiten Transistors angeordnet, wobei die Basis des zweiten Transistors von einer monostabilen Kippstufe angesteuert wird, die von am Ausgangskreis abgegebenen Impulsen aktiviert wird.

Die Erfindung beruht darauf, daß der unsymmetrisch begrenzende, normalerweise im Chip integrierte Ausgangskreis eines Herzschrittmachers durch die Aufnahme einer symmetrisch begrenzenden Schaltung von dem Anschlußklemmenpaar des Herzschrittmachers abgekoppelt wird. Diese komplette Abkopplung des unsymmetrisch begrenzenden Ausgangskreises erfolgt immer, und zwar unabhängig von evtl. vorhandenen Störsignalen, für die gesamte Impulsperiodendauer. Die symmetrisch begrenzende Abkopplungsschaltung ermöglicht die Weitergabe von Impulsen des Ausgangskreises und bewirkt die Entladung des Koppelkondensators und die der möglichen Belastungskapazitäten innerhalb einer kurzen Zeitspanne nach dem Impuls. Die Abkopplungsschaltung wird in keinster Weise über evtl. vorhandene Störsignale gesteuert. Die Störsignale gelangen somit nicht zum unsymmetrisch begrenzenden Ausgangskreis und können an der symmetrisch begrenzenden Abkopplungsschaltung nicht demoduliert werden. Eine Dämpfung dieser höherfrequenten Störsignale an dem Schrittmacher-Anschlußklemmenpaar mittels eines Kondensators zur Reduzierung dieser Demodulation am unsymmetrisch begrenzenden Ausgangskreis ist daher nicht notwendig. Die anliegenden höherfrequenten kontinuierlichen oder auch gepulsten und modulierten Störsignale von mehr als 1 kHz werden nun im Eingangskreis ordnungsgemäß stark gedämpft, so daß sie den Schrittmacher in seiner bestimmungsgemäßen therapeutischen Tätigkeit nicht beeinflussen.

Bei einer geeigneten Wahl der Schaltungselemente der Abkopplungsschaltung und des übrigen Schrittmachers kann jetzt ebenfalls auf einer Spannungsbegrenzung am Schrittmacher-Anschlußklemmenpaar verzichtet werden.

Wenn auf einer Spannungsbegrenzung am Schrittmacher-Anschlußklemmenpaar nicht verzichtet werden kann, kann eine symmetrische Eingangsspannungsbegrenzung in Form einer doppelseitigen Zenerdiode vorgenommen werden.

Gemäß einer bevorzugten Ausführungsform ist die dem Transistor vorgeschaltete Diode bezüglich ihrer Durchlaßrichtung entgegengesetzt zur Durchlaßrichtung der Kollektordiode des mit ihr verbundenen Transistors angeordnet.

Vorzugsweise liegt die Basis der Transistoren über Kondensatoren hochfrequenzmäßig auf konstanten Potential.

Gemäß einer bevorzugten Ausführungsform dient eine Anschlußklemme als Impulsausgang für den negativen Impuls, die Anode einer ersten Diode und die Kathode einer zweiten Diode sind an der Impulsausgangsklemme angeschlossen, die Kathode der ersten Diode ist mit dem Kollektor eines ersten Transistors verbunden, dessen Emitter an den Impulsausgang des Ausgangskreises angeschlossen ist und dessen Basis über einen ersten Basiswiderstand mit der anderen Anschlußklemme verbunden ist, die Anode der zweiten Diode ist mit dem Kollektor eines zweiten Transistors verbunden, dessen Emitter mit der anderen Anschlußklemme verbunden ist und dessen Basis über einen zweiten Basiswiderstand mit dem Ausgang eines Monoflops verbunden ist, das eingangsseitig mit dem Impulsausgang des Ausgangskreises in Verbindung steht, und der erste Transistor ist als npn-Transistor und der zweite Transistor als pnp-Transistor ausgebildet.

Vorzugsweise ist die Abkopplungsschaltung an der Impulsausgangsklemme mittels eines Koppelkondensators gleichspannungsmäßig abgekoppelt.

Gemäß einer bevorzugten Ausführungsform sind die Dioden Shottky-Dioden mit niedriger Vorwärtsspannung und die Transistoren haben einen hohen Stromverstärkungsfaktor.

Gemäß einer weiteren bevorzugten Ausführungsform sind die Transistoren FET-Transitoren.

Die erfindungsgemäße Schutzschaltung ist insbesondere für Herzschrittmacher aller Arten geeignet, d.h. für unipolare, bipolare und multipolare Herzschrittmacher sowie für Mehrfachkammersysteme. Die erfindungsgemäße Schutzschaltung ist insbesondere auch anwendbar bei programmierbaren, sensor- oder sensorengesteuerten und adaptiven Herzschrittmachersystemen.

Ausführungsbeispiele der Erfindung werden nachstehend an Hand der Zeichnung näher erläutert. Es zeigt:

Fig. 1 ein Schaltungsschema eines Herzschrittmachers,

Fig. 2 das Schaltbild einer Abkopplungsschaltung, und

Fig. 3 eine graphische Darstellung der Störfestigkeit bei einem Herzschrittmacher mit Abkopplungsschaltung.

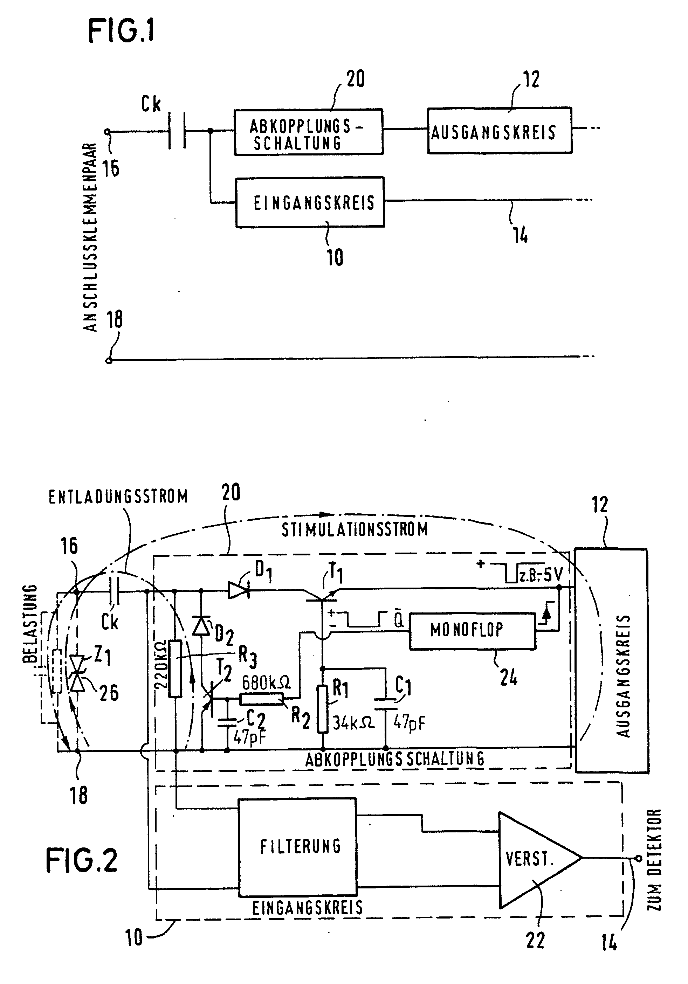

Die in Fig. 1 gezeigte Schaltung eines Herzschrittmachers weist einen Eingangskreis 10 und einen Ausgangskreis 12 auf. Der Eingangskreis 10 ist über eine Leitung 14 mit einem nicht gezeigten Detektor verbunden. Zwischen dem Ausgangskreis 12 und einem Anschlußklemmenpaar 16, 18 ist eine Abkopplungsschaltung 20 vorgesehen.

Fig. 2 zeigt die Einzelheiten der Abkopplungsschaltung 20. Die Anschlußklemme 16 dient als Stimulationsimpulsausgang für negative Stimulationsimpulse. Die Anode einer ersten Diode $D_1$ und die Kathode einer zweiten Diode $D_2$ sind an die Impulsausgangsklemme 16 angeschlossen. Die Kathode der ersten Diode $D_1$ ist mit dem Kollektor eines ersten Transistors $T_1$ verbunden, dessen Emitter an den Stimulationsimpulsausgang des Ausgangskreises 12 angeschlossen ist und dessen Basis über einen ersten Basiswiderstand $R_1$ mit der anderen Anschlußklemme (18) verbunden ist. Die Anode der zweiten Diode $D_2$ ist mit dem Kollektor eines zweiten Transistors $T_2$ verbunden, dessen Emitter mit der anderen Anschlußklemme (18) verbunden ist und dessen Basis über einen zweiten Basiswiderstand $R_2$ mit dem Ausgang eines Monoflops 24 verbunden ist. Das Monoflop 24 steht eingangsseitig mit dem Stimulationsimpulsausgang des Ausgangskreises 12 in Verbindung. Der erste Transistor $T_1$ ist als npn-Transistor und der zweite Transistor $T_2$ als pnp-Transistor ausgebildet.

Die Abkopplungsschaltung weist die zwei Dioden $D_1$ und $D_2$ auf, die jeweils mit einem Transistor $T_1$ bzw. $T_2$ in Serie geschaltet sind. Diese Schaltungen aus den Dioden und den Transistoren bewirken die vollständige Abblockung von externen Signalen jeder Art. Dies wird in jedem Zweig durch die gesperrte Kollektordiode des Transistors und durch die in Gegenrichtung vorgeschaltete Diode bewirkt. Die Kondensatoren $C_1$ und $C_2$ sorgen für die AC-Entkopplung der Basis der Transistoren $T_1$ und $T_2$. Die Aufsteuerung der beiden Transistoren über die unvermeidbaren parasitären Kapazitäten werden bei höheren Störsignalfrequenzen somit verhindert. Dadurch wird gewährleistet, daß auch höherfrequente Störsignale den asymmetrisch begrenzenden Ausgangskreis nicht erreichen. Diese werden über die parasitären Kapazitäten der beiden Dioden $D_1$, $D_2$, die Kollektorbasiskapazitäten der beiden Transistoren $T_1$ und $T_2$ und über die Kondensatoren $C_1$ und $C_2$ abgeführt. Dabei ist die Belastung dieser höherfrequenten Störsignale durch den Herzschrittmacher um ein Vielfaches geringer als bei den heutigen Schrittmacherschaltungen mit einem Kondensator (CEMI) am Schrittmacher-Anschlußklemmenpaar. Die Eingangskapazität der Abkopplungsschaltung beträgt max. 30 pF, im Gegensatz zu einem gebräuchlichen CEMI-Wert von 1 bis 10 nF.

Der negative Stimulationsimpuls des Ausgangskreises 12 wird durch den dann leitenden Transistor $T_1$ und durch die dann in Vorwärtsrichtung betriebene Diode $D_1$ zur Anschlußklemme 16 des Schrittmachers geführt. Dabei öffnet dieser Impuls selbst den Transistor $T_1$. Ist der Stimulationsimpuls mit der vom Schrittmacher bestimmten Impulsdauer zu Ende, wird durch den sofort schließenden Transistor $T_1$ die Verbindung zwischen Anschlußklemme 16 des Schrittmachers und dem Ausgangskreis 12 wieder unterbrochen.

Die Ausgangsamplitude wird lediglich durch den Spannungsabfall an dem geöffneten Transistor $T_1$ und dem Spannungsabfall an der in Vorwärtsrichtung betriebenen Diode $D_1$ reduziert. Wenn als Diode $D_1$ eine Shottky-Diode mit niedriger Vorwärtsspannung und ein Transistor ($T_1$) mit hohem Stromverstärkungsfaktor verwendet wird, läßt sich diese Ausgangsspannungsreduzierung bei einem zusätzlichen Stromverbrauch von weniger als 0,1 $\mu$A (bei 5 V Amplitude, 0,5 ms Impulsdauer, 833 ms Stimulationsperiodendauer und einem Widerstand R1 von 34 kOhm) auf ca. 0,3 V begrenzen.

Direkt nach Beendigung des Stimulationsimpulses erfolgt die Entladung des Koppelkondensators CK und die Entladung der möglichen Belastungskapazitäten über den Transistor $T_2$ und die Diode $D_2$. Der Transitor $T_2$ wird im Anschluß an den Stimulationsimpuls für eine gewisse Entladungszeit (normalerweise weniger als 100 ms) geöffnet, so daß der notwendige Entladungsstrom über $T_2$ und der nun in Vorwärtsrichtung betriebenen Diode $D_2$ fließen kann. Wenn für die Diode $D_2$ eine Shottky-Diode mit niedriger Vorwärtsspannung verwendet wird und der Transistor $T_2$ einen hohen Stromverstärkungs faktor aufweist, ist die Entladung der Kapazitäten auch bei Stimulationsimpulsen mit hoher Energie bei einem zusätzlichen Stromverbrauch von ca. 0,2 $\mu$A (bei 833 ms Stimulationsperiodendauer, 64 ms Entladungszeit und einem Widerstand $R_2$ von 680 kOhm) gewährleistet. Die während dieser Zeit (Stimulationsimpulsdauer und Entladungszeit) mögliche Demodulation von Störsignalen ist ohne Bedeutung, da der Herzschrittmacher auf jeden Fall eine absolute Refraktärzeit von mindestens 100 ms nach einem abgegebenen Stimulationsimpuls hat, während dessen evtl. anliegende Signale im Eingangskreis 10 nicht bewertet werden. Ein der Diode $D_2$ und dem Transistor $T_2$ parallel geschalteter Widerstand R3 von 220 kOhm bewirkt, sofern erforderlich, eine langsame Restentladung der Kapazitäten und bestimmt die Eingangsimpedanz des Schrittmachers maßgeblich im Niederfrequenbereich (weniger als 1 kHz).

Die Eingangssignale werden im Eingangskreis 10 ordnungsgemäß gefiltert, bevor sie zu einem Verstärker 22 im Eingangskreis gelangen. Höherfrequente Störsignale (von mehr als 1 kHz) werden durch zwei aufeinanderfolgende RC-Tiefpassfilter stark gedämpft. Die Herzsignale dagegen erreichen den Eingangsverstärker 22 fast ungedämpft.

Eine entsprechende Abkopplungsschaltung 20 kann ebenfalls mit FET-Transistoren aufgebaut werden. Dazu wird beispielsweise der Transistor $T_1$ durch einen N-Kanal MOSFET (Source am Ausgangskreis 12, Drain an Diode D1) und der Transistor $T_2$ durch einen P-Kanal MOSFET ersetzt (Source an Anschlußklemme 18, Drain an Diode $D_2$). Der Widerstand $R_1$ und der Kondensator $C_1$ können dabei entfallen, da das Gate von dem Transistor $T_1$ direkt an der Anschlußklemme 18 gelegt werden kann. Ebenso wäre es möglich, den Widerstand $R_2$ und den Kondensator $C_2$ entfallen zu lassen, da das Gate des Transistors $T_2$ direkt am Ausgang $\overline{Q}$des Monoflops 24 angeschlossen werden kann. Zudem hat diese Schaltung den Vorteil eines weitaus geringeren Stromverbrauchs, da die Basisströme der bipolaren Transistoren $T_1$ und $T_2$ entfallen.

Die Abkopplungsschaltung 20 kann insgesamt oder teilweise auf einem Schrittmacher-IC monolithisch integriert oder in den bestehenden Ausgangskreis mit aufgenommen werden, und zwar entweder diskret aufgebaut oder insgesamt bzw. teilweise monolithisch integriert.

Wenn auf eine Spannungsbegrenzung am Schrittmacher-Anschlußklemmenpaar 16, 18 nicht verzichtet werden kann, wird eine symmetrische Eingangsspannungsbegrenzung in Form einer doppelseitigen Zenerdiode 26 vorgesehen.

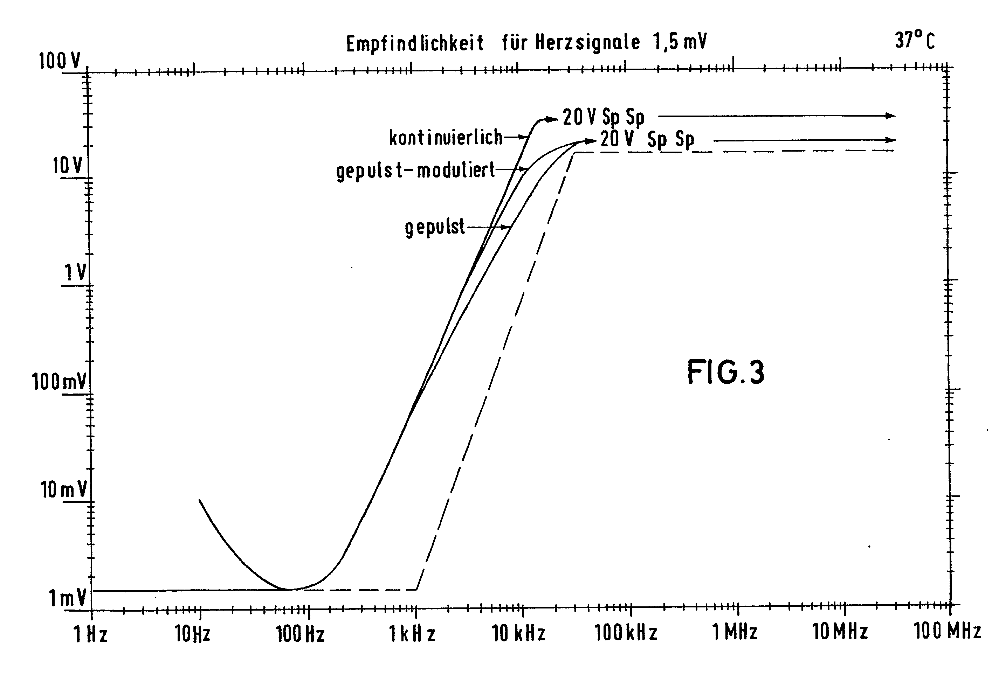

Messungen mit kontinuierlichen, gepulsten, modulierten und gepulst-modulierten (beispielsweise mit 100%-iger Amplitudenmodulation) Störsignalen an einem modernen vollintegrierten multiprogrammierbaren Herzschrittmacher (mit Telemetrie) und Abkopplungsschaltung/Eingangsschaltung, wie vorstehend beschrieben, die in Fig. 3 dargestellt sind, zeigen:

a) Eine Demodulation am Herzschrittmacher-Anschlußklemmenpaar ist über den gesamten Frequenzbereich nicht vorhanden.

b) Sämtliche höherfrequente Störsignale (auch gepulste, modulierte und gepulst-modulierte Signale) führen zu keiner Beeinflussung des Herzschrittmachers in seiner bestimmungsgemäßen therapeutischen Tätigkeit (die Beeinflussungsschwelle liegt bei mehr als 16 V für sämtliche Störsignale im Frequenzbereich von 30 kHz bis 30 MHz, ansteigend mit ca. 12 dB/Oktav von 300 Hz bis 30 kHz).

c) Die Eingangsimpedanz des Herzschrittmachers über die Frequenz bleibt relativ hochohmig und beträgt ca. 200 kOhm im Herzsignalfrequenzbereich (< 1 kHz), ca. 50 kOhm bei 10 kHz, ca. 22 kOhm bei 100 kHz und ca. 500 Ohm bei 10 MHz.

Ein Vergleich mit Herzschrittmachern ohne Abkopplungsschaltung ergibt:

a) Das niederfrequente Demodulationsprodukt am Herzschrittmacher-Anschlußklemmenpaar wird von ca. 1 V auf weniger als 1 mV reduziert (Trägerfrequenz 500 kHz, Amplitude 20 V Spitze - Spitze, Modulationsfrequenz 50 Hz, Modulationsgrad 100% AM, Generator mit 50 Ohm Ausgangsimpedanz über 100 Ohm mit Herzschrittmacher verbunden).

b) Die Störfestigkeit wird erheblich verbessert. Sie ist z.B. bei gepulsten 100% Amplitudenmodulierten Störsignalen um einen Faktor 20 besser (Trägerfrequenz zwischen 30 und 100 kHz).

c) Die Eingangsimpedanz liegt bei dem Schrittmacher mit Abkopplungsschaltung generell höher:

bei 100 Hz um einen Faktor 4 höher,
bei 10 kHz um einen Faktor 30 höher,
bei 100 kHz um einen Faktor 100 höher,
bei 10 MHz um einen Faktor 250 höher.

Die erfindungsgemäße Abkopplungsschaltung eröffnet nachstehende Möglichkeiten:

1) Mit einer erfindungsgemäßen Abkopplungsschaltung wird jeder Herzschrittmacher in seiner Störfestigkeit bezüglich Störsignalen aller Art mit Frequenzen von mehr als 1 kHz deutlich verbessert. Die Abkopplungsschaltung eignet sich auch zur Nachrüstung vorhandener Herzschrittmacher.

2) Jeder Herzschrittmacher wird durch die Anwendung der erfindungsgemäßen Abkopplungsschaltung in seiner bestimmungsgemäßen therapeutischen Tätigkeit durch die heute maximal zulässigen elektrischen und magnetischen Dauerfeldstärken ab 30 kHz nicht beeinflußt (DIN/VDE 0848, Teil 2, Entwurf August 1986).

3) Die Beeinflussungsschwelle eines Herzschrittmachers für kontinuierliche und für gepulste, modulierte und gepulst-modulierte Störsignale wird um ein Vielfaches erhöht. Durch die fehlende Demodulation am Ausgangskreis und die ordnungsgemäße starke Dämpfung dieser Störsignale gegenüber dem Herzsignal im Eingangskreis arbeitet ein mit der Abkopplungsschaltung versehener Herzschrittmacher auch bei Vorhandensein von kontinuierlichen, gepulsten, modulierten und gepulst-modulierten Störsignalen unbeeinflußt (siehe Fig. 3).

4) Eine Demodulation von Störsignalen aller Art ist an dem Anschlußklemmenpaar eines Herzschrittmachers mit Abkopplungsschaltung über den gesamten Frequenzbereich ausgeschlossen.

5) Das durch die Demodulation von Störsignalen am Herzschrittmacher-Anschlußklemmenpaar induzierbare Herzflimmern ist somit ausgeschlossen.

6) Die Eingangsimpedanz (als Funktion der Frequenz) eines Herzschrittmachers kann durch das Entfallen oder die Reduzierung der Kapazität direkt am Herzschrittmacher-Anschlußklemmenpaar drastisch erhöht werden.

7) Die Größe des am Herzschrittmacher-Anschlußklemmenpaar fließenden Störstroms wird durch die Erhöhung der Eingangsimpedanz drastisch reduziert.

8) Die Gefahr einer Gewebsverbrennung ist durch diese drastische Reduzierung des Störstroms ausgeschlossen.

9) Alle vorstehend angeführten Punkte gelten für Implantate, die einen Ausgangskreis und einen Eingangskreis für die Aufnahme von therapeutisch und/oder diagnostisch bedeutsamen niederfrequenten Herzsignalen besitzen.

10) Abgesehen von der unter Punkt 5 getroffenen Aussage gelten vorstehend angeführte Punkte für Implantate, die einen Ausgangskreis und einen Eingangskreis für die Aufnahme von therapeutisch und/oder diagnostisch bedeutsamen niederfrequenten Signalen besitzen.

11) Die unter Punkt 4) und 5) getroffenen Aussagen gelten für Implantate, die einen Ausgangskreis besitzen und mit dem Herzen verbunden sind.

12) Die unter Punkt 4) getroffene Aussage ist aufrechtzuerhalten bei Implantaten, die einen Ausgangskreis besitzen.

13) Die Punkte 1) bis 8) gelten für externe therapeutisch und/oder diagnostisch wirksame Geräte, die einen mit dem Herzen verbundenen Ausgangs- und Eingangskreis für die Aufnahme von therapeutisch und/oder diagnostisch bedeutsamen niederfrequenten Herzsignalen besitzen.

14) Mit Ausnahme des unter Punkt 5) Genannten, gelten die Aussagen der Punkte 1) bis 8) für externe therapeutisch und/oder diagnostisch wirksame Geräte, die einen mit dem Körper verbundenen Ausgangs- und Eingangskreis für die Aufnahme von therapeutisch und/oder diagnostisch bedeutsamen niederfrequenten Signalen besitzen.

15) Die unter Punkt 4) und 5) getroffenen Aussagen gelten für externe therapeutisch und/oder diagnostisch wirksame Geräte, die einen mit dem Herzen verbunden Ausgangskreis besitzen.

16) Eine Demodulation von Störsignalen aller Art ist an dem Anschlußklemmenpaar eines externen therapeutisch und/oder diagnostisch wirksamen Gerätes, das einen mit dem Körper verbundenen Ausgangskreis mit Abkopplungsschaltung besitzt, über den gesamten Frequenzbereich ausgeschlossen.

## Ansprüche

1. Schutzschaltung zur Beseitigung des Einflusses von kontinuierlichen, gepulsten oder modulierten Störsignalen für therapeutisch und/oder diagnostisch wirksame Geräte, insbesondere für Herzschrittmacher dadurch **gekennzeichnet,** daß zwischen Ausgangskreis (12) und dem Anschlußklemmenpaar (16, 18) eine Abkopplungsschaltung (20) angeordnet ist, die eine erste Serienschaltung aus einer Diode ($D_1$) und der Kollektor-Emitterstrecke eines Transistors ($T_1$) zwischen einem Ausgangskreis (12) und einer Anschlußklemme (16) enthält, wobei die Basis des Transistors ($T_1$) über einen Basiswiderstand ($R_1$) mit der anderen Anschlußklemme (18) verbunden ist.

2. Schutzschaltung nach Anspruch 1, dadurch **gekennzeichnet,** daß zwischen den beiden Anschlußklemmen (16, 18) eine zweite Serienschaltung aus einer Diode ($D_2$) und der Kollektor-Emitterstrecke eines zweiten Transistors ($T_2$) angeordnet ist, wobei die Basis des zweiten Transistors ($T_2$) von einer monostabilen Kippstufe angesteuert wird, die von am Ausgangskreis (12) abgegebenen Impulsen aktiviert wird.

3. Schutzschaltung nach einem der Ansprüche 1 oder 2, dadurch **gekennzeichnet,** daß die dem Transistor vorgeschaltete Diode bezüglich ihrer Durchlaßrichtung entgegengesetzt zur Durchlaßrichtung der Kollektordiode des mit ihr verbundenen Transistors angeordnet ist.

4. Schutzschaltung nach einem der vorhergehenden Ansprüche, dadurch **gekennzeichnet,** daß die Basis der Transistoren ($T_1$, $T_2$) über Kondensatoren ($C_1$, $C_2$) hochfrequenzmäßig auf konstantem Potential liegt.

5. Schutzschaltung nach einem der vorhergehenden Ansprüche, dadurch **gekennzeichnet,** daß die Anschlußklemme (16) als Impulsausgang für einen negativen Impuls dient, daß die Anode einer ersten Diode ($D_1$) an der Impulsausgangsklemme (16) angeschlossen ist, und daß die Kathode der ersten Diode ($D_1$) mit dem Kollektor eines ersten Transistors ($T_1$) verbunden ist, dessen Emitter an den Impulsausgang des Ausgangskreises (12) angeschlossen ist und dessen Basis über einen ersten Basiswiderstand ($R_1$) mit der anderen Anschlußklemme (18) verbunden ist, und daß der erste Transistor ($T_1$) als npn-Transistor ausgebildet ist.

6. Schutzschaltung nach Anspruch 5, dadurch **gekennzeichnet,** daß die Kathode einer zweiten Diode ($D_2$) an der Impulsausgangsklemme (16) angeschlossen ist, und daß die Anode dieser zweiten Diode ($D_2$) mit dem Kollektor eines zweiten Transistors ($T_2$) verbunden ist, dessen Emitter mit der anderen Anschlußklemme (18) verbunden ist und dessen Basis über einen zweiten Basiswiderstand ($R_2$) mit dem Ausgang eines Monoflops (24) verbunden ist, das eingangsseitig mit dem Impulsausgang des Ausgangskreises (12) in Verbindung steht, und daß der zweite Transistor ($T_2$) als pnp-Transistor ausgebildet ist.

7. Schutzschaltung nach einem der vorhergehenden Ansprüche, dadurch **gekennzeichnet,** daß die Anschlußklemme (16) als Impulsausgang für einen positiven Impuls dient, daß die Kathode einer ersten Diode ($D_1$) an der Impulsausgangsklemme (16) angeschlossen ist, und daß die Anode der ersten Diode ($D_1$) mit dem Kollektor eines ersten Transistors ($T_1$) verbunden ist, dessen Emitter an den Impulsausgang des Ausgangskreises (12) angeschlossen ist und dessen Basis über einen ersten Basiswiderstand ($R_1$) mit der anderen Anschlußklemme (18) verbunden ist, und daß der erste Transistor ($T_1$) als pnp-Transistor ausgebildet ist.

8. Schutzschaltung nach Anspruch 7, dadurch **gekennzeichnet,** daß die Anode einer zweiten Diode ($D_2$) an der Impulsausgangsklemme (16) angeschlossen ist, und daß die Kathode dieser zweiten Diode ($D_2$) mit dem Kollektor eines zweiten Transistors ($T_2$) verbunden ist, dessen Emitter mit der anderen Anschlußklemme (18) verbunden ist und dessen Basis über einen zweiten Basiswiderstand ($R_2$) mit dem Ausgang eines Monoflops (24) verbunden ist, das eingangsseitig mit dem Impulsausgang des Ausgangskreises (12) in Verbindung steht, und daß der zweite Transistor ($T_2$) als npn-Transistor ausgebildet ist.

9. Schutzschaltung nach einem der vorhergehenden Ansprüche, dadurch **gekennzeichnet,** daß die Abkopplungsschaltung (20) an der Anschlußklemme (16) mittels eines Koppelkondensators (Ck) gleichspannungsmäßig abgekoppelt ist.

10. Schutzschaltung nach einem der vorhergehenden Ansprüche, dadurch **gekennzeichnet,** daß die Dioden ($D_1$, $D_2$) Shottky-Dioden mit niedriger Vorwärtsspannung sind und die Transistoren ($T_1$, $T_2$) einen hohen Stromverstärkungsfaktor haben.

11. Schutzschaltung nach einem der vorhergehenden Ansprüche, dadurch **gekennzeichnet,** daß die Transistoren FET-Transistoren sind.

12. Schutzschaltung nach einem der vorhergehenden Ansprüche, dadurch **gekennzeichnet,** daß zwischen dem Anschlußklemmenpaar (16, 18) eine symmetrische Eingangsspannungsbegrenzung vorhanden ist.

13. Schutzschaltung nach Anspruch 12, dadurch **gekennzeichnet,** daß die Eingangsspannungsbegrenzung in Form einer doppelseitigen Zenerdiode (26) ausgebildet ist.

14. Schutzschaltung nach einem der vorhergehenden Ansprüche, dadurch **gekennzeichnet,** daß die Schaltung insgesamt oder teilweise monolithisch integriert ist.

15. Schutzschaltung nach einem der vorhergehenden Ansprüche, dadurch **gekennzeichnet,** daß die Schaltung in dem Ausgangskreis mitaufgenommen wird, und zwar entweder diskret aufgebaut oder insgesamt bzw. teilweise monolithisch integriert.

## FIG.1

## FIG.2

Empfindlichkeit für Herzsignale 1,5 mV    37°C

kontinuierlich → 20 V Sp Sp

gepulst-moduliert → 20 V Sp Sp

gepulst →

FIG.3

EP 0 426 969 A2